# FASCICULE DE BREVET EUROPEEN

(11) **EP 2 205 496 B1**
(45) Date de publication et mention de la délivrance du brevet: **24.08.2011**
(21) Numéro de dépôt: 08842641.6
(22) Date de dépôt: 01.10.2008
(51) Int. Cl.: B65D 30/20, C12M 1/02, A61J 1/05, B01F 7/16, B01F 15/00

(54) **Récipient de traitement d'un produit biopharmaceutique comprenant des moyens de protection, dispositif et procédé**
Gefäss zur Verarbeitung eines biopharmazeutischen Produkts mit Schutzmittel, Einrichtung und Verfahren
Vessel for processing a biopharmaceutical product including protection means, device and method

(30) Priorité: 04.10.2007 FR 0706974
(43) Date de publication de la demande: 14.07.2010
(73) Titulaire: Sartorius Stedim Biotech, 13781 Aubagne (FR)
(72) Inventeur: BERNARD, Frédéric, F-83740 La Cadiere d'Azur (FR); CHEVALIER, Eric, F-75015 Paris (FR)
(74) Mandataire: Derambure, Christian
(86) Numéro de dépôt international: PCT/FR2008/051768
(87) Numéro de publication internationale: WO 2009/053572

(56) Documents cités:
- GB-A- 2 080 756
- US-A- 5 350 080
- US-A1- 2007 224 676

## Description

L'invention concerne un récipient de traitement d'un produit biopharmaceutique. Elle vise plus particulièrement un tel récipient, un dispositif de traitement incorporant un tel récipient et le procédé de mise en oeuvre d'un tel récipient.

On entend ici par « produit biopharmaceutique », un (ou plusieurs) produit issu de la biotechnologie - milieux de cultures, cultures cellulaires, solutions tampon, liquides de nutrition artificielle, produits sanguins et dérivés de produits sanguins - ou un produit pharmaceutique ou plus généralement un produit destiné à être utilisé dans le domaine médical. Un tel produit est sous forme liquide, pâteuse, poudreuse, en une ou plusieurs phases, homogènes ou non. L'invention s'applique également à des produits autres que biopharmaceutiques, selon la définition qui vient d'en être donnée, mais qui soumis à des exigences analogues en ce qui concerne leur traitement.

On connaît une poche destinée à recevoir un tel produit biopharmaceutique, qui comporte une paroi d'extrémité inférieure, une paroi d'extrémité supérieure et, entre elles, une paroi latérale souple pouvant se trouver - à l'instar de la poche elle-même - dans deux états extrêmes - un état plié à plat et un état déplié déployé - et être déformée pour passer de l'un à l'autre de ces états extrêmes ou être dans tout état intermédiaire. Les parois de la poche, en matière plastique telle que le polyéthylène ou un complexe comprenant du polyéthylène, délimitent un espace interne qui, à l'état plié, est de volume minimal, voire nul ou quasi nul, et, à l'état déplié et déployé est maximal. Cet espace est destiné à recevoir le produit biopharmaceutique.

Une telle poche est destinée soit au simple stockage du produit biopharmaceutique soit à un traitement spécifique, c'est-à-dire toute action sur le produit, ou concernant ce produit, et impliquant un contact avec le produit, telle que mélange, aération, filtration, mesure, cette liste n'étant pas limitative. A cette fin, on met en oeuvre tel moyen de traitement adapté à l'action souhaitée, tel que hélice de mélange, aérateur, filtre, sonde, cette liste n'étant pas non plus limitative. Un tel (ou de tels) moyen de traitement est, au moins pour partie, placé à l'intérieur même de la poche en étant adjacent à sa partie inférieure (sur elle, proche d'elle ou au voisinage d'elle), de manière à pouvoir être en contact avec le produit.

Les dispositions constructives précédentes ne sont pas sans risque. En effet, lorsque la poche est à l'état plié à plat ou dans un état légèrement déplié ou presque plié ou passe de l'un à l'autre de ces états, il existe un risque que la partie interne active du moyen de traitement n'interfère avec la paroi de la poche, plus spécialement la paroi d'extrémité supérieure et altère plus ou moins gravement son intégrité et ses qualités, en la fragilisant, en la perforant ou en la déchirant. Par ailleurs, il existe également un risque que la partie interne active du moyen de traitement n'interfère avec des éléments exogènes et extérieurs à la poche (tels que des supports ou d'autres dispositifs se trouvant à côté de la poche) et s'en trouve altérée plus ou moins sérieusement, par exemple étant déformée ou déplacée ou encore coincée.

Ces dispositions constructives présentent un autre inconvénient, à savoir ne pas permettre un vidage complet du contenu de la poche. En effet, la poche sous l'effet de la diminution de la pression résultant du vidage, se plaque contre la partie interne active du moyen de traitement, avec un risque sérieux de déchirement, le plus souvent pallié en ne vidant pas complètement la poche.

Le document WO 00/04131 décrit une poche à soufflets ayant une partie latérale, une partie supérieure et une partie inférieure, destinée à contenir un (ou plusieurs) tel produit biopharmaceutique, de volume de 50 litres et plus. Une telle poche peut se trouver soit dans un état plié à plat pour être stockée vide soit dans un état déplié et déployé où elle a une forme générale parallélépipédique lorsqu'elle contient du produit. Selon les réalisations, une telle poche comprend également, en partie supérieure, un (ou des) orifice ou connexion destiné à permettre son remplissage et, en partie inférieure, une bonde destinée à permettre sa vidange.

Le document US 5 350 080 décrit un système selon le préambule de la revendication 1 comprenant un conteneur extérieur rigide supportant une poche définissant une chambre interne de stockage dont l'accès est possible à travers un port de remplissage situé en partie supérieure, un port de vidange pouvant être prévu en partie inférieure. Lorsque le contenu de la poche est vidangé, le port de remplissage supérieur est entraîné par son propre poids vers la partie inférieure de la poche. Dans le cas où la partie interne du port de remplissage est dépourvue de tube, elle peut venir au contact de la partie inférieure de la poche avec le risque de l'endommager et de venir se bloquer. Aussi, il est prévu que le port de remplissage comporte une jupe latérale dont la hauteur est plus grande que la longueur en saillie vers le bas de la partie interne du port de remplissage, de sorte que cette partie interne est toute entière logée dans la jupe et que son extrémité inférieure est toujours située au dessus de la partie inférieure de la poche, même quand la jupe repose sur celle-ci.

Les documents US 2007/0224676 et GB 2 080 756 décrivent des récipients comportant une paroi latérale souple en accordéon, pouvant être pliée ou dépliée et, de part et d'autre de celle-ci, deux parois d'extrémité, respectivement, inférieure et supérieure, cette dernière pourvue d'une ouverture.

L'invention vise à pallier les inconvénients susmentionnés liés à la présence de moyens de traitement disposés dans des poches pliables aptes à être disposées pliées à plat.

A cet effet, et selon un premier aspect, l'invention vise un récipient de traitement d'au moins un produit biopharmaceutique, comprenant :
■ une poche ayant une paroi latérale souple, une partie d'extrémité inférieure et une partie d'extrémité supérieure délimitant un espace interne destiné à recevoir le au moins un produit, pouvant se trouver dans deux états extrêmes, plié à plat et déployé, et dans tout état intermédiaire, et être déformée pour passer d'un état à un autre ;
■ des moyens de traitement du au moins un produit contenu dans l'espace de réception de la poche, comportant une partie interne d'une certaine hauteur disposée à l'intérieur du récipient;
■ des moyens de protection des moyens de traitement, comportant une paroi latérale, une partie d'extrémité inférieure et une partie d'extrémité supérieure délimitant un espace interne en communication avec l'espace interne de la poche, dont la partie d'extrémité inférieure est attenante et fixée rigidement à la partie d'extrémité supérieure de la poche, le déplacement de celle-ci vers le haut ou le bas entraînant un déplacement des moyens de protection, la partie interne des moyens de traitement étant essentiellement disposée dans l'espace interne des moyens de protection lorsque la poche est à l'état plié à plat, la partie périphérique de l'espace interne des moyens de protection autour des moyens de traitement formant une chambre de protection de la poche et des moyens de traitement ;
■ au moins un orifice d'introduction dans le récipient du au moins un produit et/ou de
   vidage du récipient de son contenu ;
   dans lequel :
■ les moyens de traitement sont au moins pour partie - organe de mélange, capteur, tube, organe d'aération ou autre - disposés à l'intérieur de la poche en étant adjacents à sa partie d'extrémité inférieure ;
■ les moyens de traitement et les moyens de protection sont agencés pour être indépendants l'un de l'autre en ce qui concerne leurs déplacements ;
■ la hauteur de la chambre de protection, variable, peut être l'une de deux hauteurs extrêmes, maximale et minimale, et toute hauteur intermédiaire, et s'adapter automatiquement, en fonction de l'état respectivement plié à plat, déplié et déployé, et tout état intermédiaire, de la poche;
   de manière que dans chaque état de la poche, les moyens de traitement sont écartés de la paroi latérale et de la partie d'extrémité supérieure de la poche, tout en étant adjacents à sa partie d'extrémité inférieure.

Dans une première réalisation, les moyens de protection sont rigides et indéformables. Dans une seconde réalisation, les moyens de protection sont déformables en hauteur, la paroi latérale comprenant au moins un tronçon déformable entre deux états extrêmes, respectivement plié et déployé, apte à être déformé pour passer d'un état à l'autre ; le récipient comprenant en outre des moyens aptes à maintenir le tronçon déformable déployé, au moins à l'état extrême plié à plat de la poche. Dans cette seconde réalisation, ces moyens aptes à maintenir le tronçon déformable déployé, au moins à l'état extrême plié à plat de la poche, sont soit intégrés à la paroi latérale des moyens de protection soit distincts d'elle.

Selon une réalisation, les moyens de protection sont agencés pour former une tête d'introduction d'au moins un produit dans le récipient. Dans ce cas, les moyens de protection formant une tête d'introduction comprennent au moins un orifice d'introduction du dit au moins produit ménagé dans la partie d'extrémité supérieure des moyens de protection et des moyens d'obturation amovible du dit orifice aptes à assurer sa fermeture et son ouverture, tel qu' un bouchon ou une membrane. En outre, la tête d'introduction peut, dans une réalisation, comprendre des moyens d'association amovible du récipient à un dispositif de transfert aseptique.

Selon une réalisation, il est prévu au moins un orifice d'introduction dans le récipient d'au moins un produit et/ou de vidage du récipient de son contenu ménagé sur la poche, notamment dans la partie d'extrémité inférieure.

Selon une réalisation, il est prévu une poche garnie à l'intérieur d'une plaque rigide sur sa partie d'extrémité inférieure.

Selon une caractéristique, les moyens de protection assurent en combinaison la fonction de protection de la poche et des moyens de traitement et une fonction de guidage de la poche lorsque, l'orifice de remplissage du récipient étant ouvert, on introduit progressivement dans le récipient le au moins un produit, alors que simultanément la poche se déploie vers le haut.

Selon une réalisation, les moyens de traitement et les moyens de protection sont dissociés structurellement les uns des autres, étant distincts les uns des autres et sans contact direct entre eux, la partie périphérique de l'espace interne des moyens de protection autour des moyens de traitement formant une chambre de protection s'étendant latéralement et supérieurement entre les moyens de traitement et la paroi latérale et la partie d'extrémité supérieure des moyens de protection.

Selon une réalisation, les moyens de traitement ont une partie à l'intérieur de la poche qui, à l'état plié à plat de la poche, occupe substantiellement l'espace interne des moyens de protection. Selon une réalisation, les moyens de traitement ont une partie à l'intérieur de la poche qui, à l'état déployé de la poche, se trouve substantiellement intégralement dans l'espace interne de la poche, la hauteur, ici minimale, de la chambre de protection étant nulle.

Selon une réalisation, les moyens de traitement ont une partie à l'intérieur de la poche portée, le cas échéant avec possibilité de déplacement relatif, par la face intérieure de la partie d'extrémité inférieure de la poche ou par une plaque rigide garnissant la partie d'extrémité inférieure de la poche à l'intérieur.

Selon une caractéristique, le récipient comporte des moyens aptes à participer à son maintien dans un conteneur, la poche contenant la quantité souhaitée du au moins un produit. Selon une réalisation, de tels moyens sont intégrés ou associés aux moyens de protection.

Selon une application particulière, les moyens de traitement sont des moyens de mélange, le récipient comportant au moins un premier orifice d'introduction dans le récipient d'une première partie d'au moins un produit ménagé sur la poche, notamment adjacent à sa partie d'extrémité inférieure et au moins un second orifice d'introduction d'une seconde partie d'au moins un produit ménagé dans la partie d'extrémité supérieure des moyens de protection. Le récipient est alors un récipient de mélange. Selon un deuxième aspect, l'invention vise un dispositif de traitement d'au moins un produit biopharmaceutique, comprenant un récipient tel qu'il vient d'être décrit et un conteneur rigide apte à recevoir le récipient dont la poche est à l'état plié à plat, à l'état déployé, ou dans un état intermédiaire, à permettre à la poche d'être déformée pour passer d'un état à un autre, et à guider la poche, notamment sa paroi latérale souple, afin que la poche soit mise en volume en ayant un volume complémentaire de celui du conteneur, lorsque l'on introduit dans le récipient le au moins un produit.

Selon un autre aspect, l'invention vise un procédé de mise en oeuvre d'un tel dispositif de traitement, dans lequel :
■ on dispose d'un récipient et d'un conteneur constitutifs du dispositif de traitement et on place la poche du récipient à l'état plié à plat dans le fond du conteneur ;
■ un orifice d'introduction de produit du récipient étant ouvert, on introduit progressivement dans le récipient le au moins un produit et simultanément on laisse la poche se déployer vers le haut et être mise en volume par le conteneur, l'espace interne de la poche, de volume croissant, recevant le au moins un produit ; on poursuit cette étape jusqu'à avoir introduit dans le récipient la quantité souhaitée du au moins un produit ;
■ durant cette étape, on laisse les moyens de protection se déplacer vers le haut étant entraînés par le déplacement de la partie d'extrémité supérieure de la poche ;
■ durant cette étape, on laisse la hauteur de la chambre de protection diminuer à partir de la hauteur extrême maximale et s'adapter automatiquement en fonction de l'état de la poche jusqu'à ce que la poche soit dans l'état déplié et déployé ou tout état intermédiaire en fonction de la quantité de produit introduite dans le récipient, de manière que dans tous les états de la poche, les moyens de traitement soient écartés de la paroi latérale et de la partie d'extrémité supérieure de la poche, tout en étant adjacents à sa partie d'extrémité inférieure :
■ on met en oeuvre les moyens de traitement du au moins un produit se trouvant dans le récipient.

Selon les réalisations, on commence la mise en oeuvre des moyens de traitement du au moins un produit se trouvant dans le récipient avant, au moment ou après avoir introduit dans le récipient la quantité souhaitée du au moins un produit.

Selon une application particulière au mélange :
■ on dispose d'un récipient de mélange dont le au moins un second orifice est obturé ;
■ on place la poche du récipient à l'état plié à plat dans le fond du conteneur ;
■ on introduit dans le récipient de mélange une première partie d'au moins un produit par au moins un premier orifice d'introduction ouvert à cette fin ;
■ et on introduit dans le récipient une seconde partie d'au moins un produit par au moins un second orifice d'introduction ouvert à cette fin.

Selon les réalisations de ce procédé de mélange, on met en oeuvre les moyens de mélange alors que le au moins un second orifice d'introduction est ouvert ou fermé.

Selon une réalisation, rendue possible par l'invention, on met en oeuvre les moyens de mélange jusqu'à vidage complet ou substantiellement complet de la poche.

On décrit maintenant plusieurs modes de réalisation de l'invention à l'aide des dessins, dans lesquels :
- la figure 1 est une vue schématique en coupe axiale d'un premier mode de réalisation d'un dispositif de traitement - ici de mélange - ayant un récipient avec moyens de protection rigides et indéformables, le récipient de mélange, dont la poche est à l'état extrême plié à plat, comportant une tête d'introduction d'au moins un produit, ayant un orifice et des moyens d'obturation fermant le récipient ;
- la figure 2 est une vue schématique en coupe axiale du récipient de mélange de la figure 1 pourvu d'autres moyens d'obturation ;
- la figure 3 est une vue schématique en coupe axiale du dispositif de mélange de la figure 1, la poche étant à l'état extrême déployé ;
- la figure 4 est une vue schématique en coupe axiale d'un second mode de réalisation du récipient, avec moyens de protection déformables, le récipient de traitement - ici de mélange - dont la poche est à l'état extrême plié à plat, comportant une tête d'introduction d'au moins un produit, ayant un orifice et des moyens d'obturation fermant le récipient ; et
- la figure 5 est une vue schématique en coupe axiale du récipient de la figure 4, la poche étant à l'état extrême déployé.

Un dispositif 1 de traitement d'au moins un produit biopharmaceutique, comprend un récipient 2 de traitement et un conteneur 3 rigide apte à recevoir le récipient 2, de façon amovible.

Comme indiqué plus haut, on entend par « produit biopharmaceutique », un (ou plusieurs) produit issu de la biotechnologie - milieux de cultures, cultures cellulaires, solutions tampon, liquides de nutrition artificielle, produits sanguins et dérivés de produits sanguins - ou un produit pharmaceutique ou plus généralement un produit destiné à être utilisé dans le domaine médical.

Un tel produit est sous forme liquide, pâteuse, poudreuse, en une ou plusieurs phases, homogènes ou non.

Bien entendu, l'invention s'applique également à des produits autres que biopharmaceutiques, selon la définition qui vient d'en être donnée, mais qui soumis à des exigences analogues en ce qui concerne leur traitement.

Comme indiqué plus haut, on entend par « traitement » toute action sur le produit biopharmaceutique, ou concernant ce produit, et impliquant un contact avec le produit.

Un tel traitement est dans la réalisation particulière décrite par la suite le mélange.

Dans d'autres réalisations, le traitement est l'aération, la filtration, la mesure, cette liste n'étant pas limitative.

Dans tous les cas, on met en oeuvre des moyens 4 de traitement adaptés à l'action souhaitée.

Par exemple, les moyens 4 comportent une partie active interne 5, rigide dans son ensemble et, selon les cas, déformable ou non. Une telle partie active interne 5 est par exemple une hélice dans le cas du mélange, un aérateur dans le cas de l'aération, un filtre dans le cas de la filtration, une sonde dans le cas de la mesure, cette liste n'étant pas davantage limitative.

Dans le cas où le traitement est la mesure, celle-ci est faite à des fins diverses : contrôle de tel paramètre concernant le produit biopharmaceutique, commande du traitement...

Le récipient 2 comprend, outre les moyens de traitement 4, une poche 6 et des moyens 7 de protection assurant une première fonction de protection de la poche 6 et des moyens 4 de traitement.

Dans la forme de réalisation représentée et décrite, le conteneur 3 a une forme générale parallélépipédique, notamment cubique ou sensiblement cubique, ayant un fond 8, une paroi latérale 9 comprenant quatre panneaux et une ouverture 10 opposée au fond 8, limitée par un retour 10a en collerette de la paroi latérale 9. Il est entendu que le conteneur 3 peut avoir une autre forme, si nécessaire.

Dans la position normale dans laquelle le conteneur 3 peut être utilisé, le fond 8 s'étend horizontalement en partie inférieure, la paroi latérale 9 s'étend verticalement et l'ouverture 10 est placée en partie supérieure à l'aplomb du fond 8. D'autre part, la paroi latérale 9 peut comporter une porte d'accès et le fond 8 et/ou la paroi latérale des orifices ou passages de dispositifs associés au récipient 2 tels que des connections.

Les mots « horizontal », « inférieur », « vertical », « supérieur », comme les mots « hauteur », « haut », « bas », se réfèrent au dispositif 1 de traitement, au récipient 2 et au conteneur 3 se trouvant dans leur position normale dans laquelle ils peuvent être utilisés. Il est bien entendu néanmoins qu'ils peuvent être placés dans une autre position, dans d'autres circonstances.

Il est entendu, en outre, que les mots « horizontal » et « vertical » visent à la fois les positions respectives exactement horizontale et verticale et celles qui le sont sensiblement, sans qu'il soit nécessaire de le préciser expressément.

On décrit maintenant plus spécialement la poche 6.

La poche 6 comprend une paroi latérale 11, souple, une partie d'extrémité inférieure 12, plane, et une partie d'extrémité supérieure 13, délimitant un espace interne 14 destiné à recevoir le au moins un produit.

Lorsque, se trouvant dans le conteneur 3, la poche 6 est déployée et mise en volume, elle présente, dans la réalisation représentée et décrite, une forme générale parallélépipédique, notamment cubique ou sensiblement cubique, comme le conteneur 3, avec une paroi latérale 11 disposée verticalement, une partie d'extrémité inférieure 12 disposée horizontalement et une partie d'extrémité supérieure 13 disposée elle-aussi horizontalement ou plus ou moins inclinée en forme de toit. Il est entendu que la poche 6 peut avoir une autre forme, si nécessaire, en correspondance avec celle du conteneur 3.

La partie d'extrémité supérieure 13 comporte un ou plusieurs passages ou ouvertures 15. Dans la réalisation représentée et décrite, il est prévu un passage unique 15 situé dans la partie médiane de la partie d'extrémité supérieure 13, laquelle présente une forme générale de collerette ou d'anneau.

Une telle poche 6 peut avoir un volume qui dépasse 50 l est atteindre 3.000 l. Elle peut avoir une longueur, une largeur et une hauteur comprise, selon le volume, entre une trentaine et près de cent cinquante centimètres.

Dans une réalisation, la paroi latérale 11, la partie d'extrémité inférieure 12 et la partie d'extrémité supérieure 13 de la poche 6 sont réalisées d'un seul tenant et entièrement en matière plastique, à partir d'un matériau en feuille assez mince pour présenter la souplesse requise. Ce matériau est choisi en fonction du contenu que la poche est destinée à recevoir et, en particulier, il est biocompatible avec les produits biopharmaceutiques reçus, apte à la stérilisation et imperméable aux gaz.

Typiquement, le matériau constitutif de la poche 6 est tel que la poche 6 est susceptible d'être fragilisée, perforée ou déchirée en cas d'interférence avec quelque chose de rigide présentant des angles, des coins, des pointes, des bords tranchants, ce qui peut être le cas de la partie interne active 5, rigide des moyens 4 de traitement.

Symétriquement, cette partie interne active 5, rigide, si elle comporte des angles, des coins, des pointes, des bords tranchants, peut être altérée plus ou moins sérieusement, par exemple déformée ou déplacée ou encore coincée, en cas d'interférence avec quelque chose de rigide ou en cas de choc, le matériau constitutif de la poche 6 n'étant pas apte en soi à la protéger suffisamment.

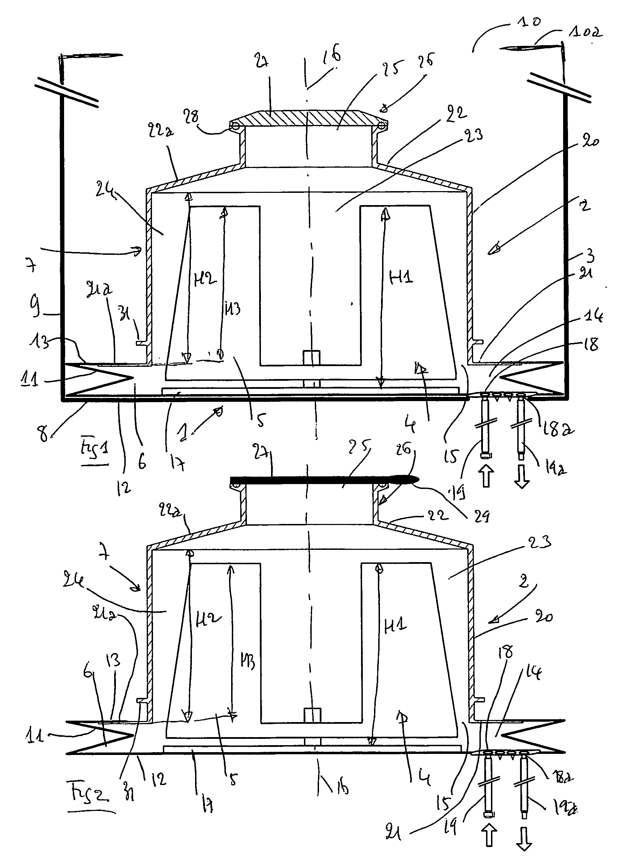

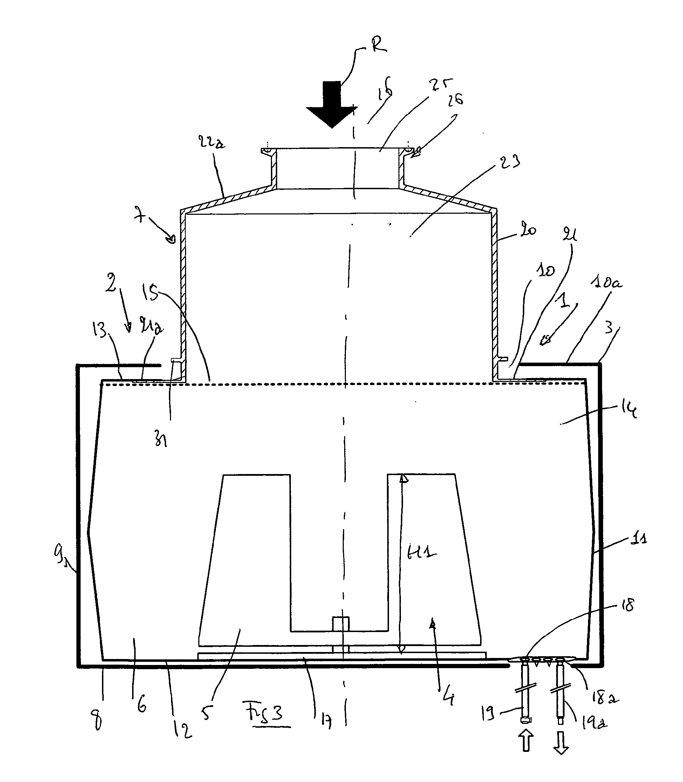

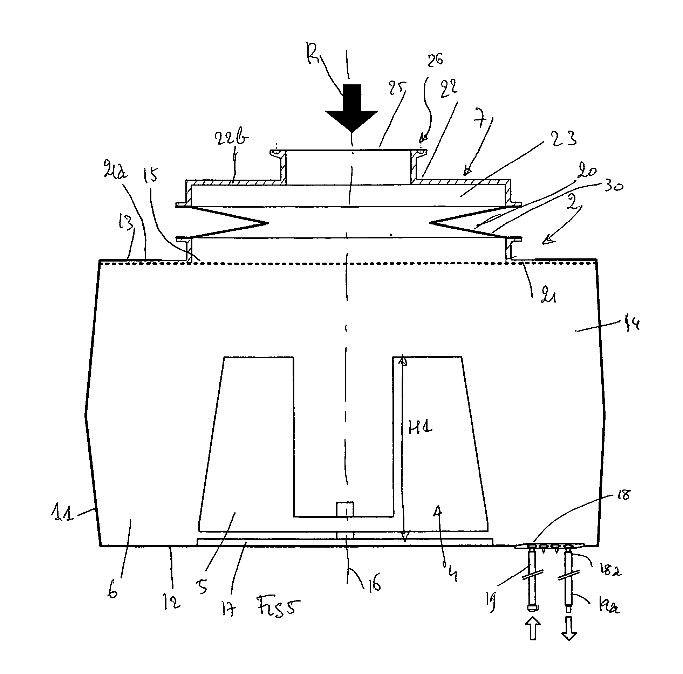

La paroi latérale 11 souple est telle que la poche 6 peut se trouver dans deux états extrêmes, à savoir un état plié à plat (figures 1, 2 et 4) et un état déployé (figures 3 et 5). La poche 6 peut également se trouver dans tout état intermédiaire entre les deux états extrêmes, à savoir partiellement déployé. Par ailleurs, la poche 6, plus spécialement la paroi latérale 11, peut être déformée pour passer d'un état à un autre, notamment lorsqu'on l'emplit ou lorsqu'on la vide.

Aux états plié, déployé et intermédiaire de la poche 6 correspondent des états analogues du récipient 2 qui incorpore la poche 6.

A l'état plié, la paroi latérale 11 est repliée sur elle-même de façon plus ou moins régulière en accordéon, la partie d'extrémité supérieure 13 reposant sur ou étant située au voisinage immédiat de la partie d'extrémité inférieure 12, plane. L'espace interne 14 est alors de volume minimal, voire nul ou quasi nul.

A l'état déployé, la paroi latérale 11 s'étend verticalement de façon prismatique ou cylindrique, la partie d'extrémité supérieure 13 étant écartée en hauteur par rapport à la partie d'extrémité inférieure 12, plane. L'espace interne 14 est alors de volume maximal.

La souplesse de la paroi latérale 11 est telle qu'en l'absence de sollicitation, notamment due à la présence d'un contenu dans la poche 6, elle-même placée dans le conteneur 3, la poche 6 se trouve à l'état plié ou tende vers cet état.

La poche 6, plus généralement le dispositif 1, présente un axe vertical 16, autour duquel est située la paroi latérale 11, et perpendiculairement auquel est disposée la partie d'extrémité inférieure 12.

La hauteur dont il est question est comptée le long de l'axe 16.

Dans la réalisation représentée et décrite, il est prévu une plaque rigide 17 disposée dans la poche 6 sur la face interne de sa partie d'extrémité inférieure 12.

Dans la réalisation représentée et décrite, il est ménagé sur la poche 6 un premier orifice 18 d'introduction dans la poche 6 (et donc le récipient 2) d'une première partie d'au moins un produit à mélanger.

Ce premier orifice 18 est adjacent à la partie d'extrémité inférieure 12, par exemple plus ou moins latéralement. S'il est prévu une plaque rigide 17 et si l'orifice 18 est en regard de celle-ci, l'orifice 18 est ménagé également dans la plaque rigide 17.

A l'orifice 18 peuvent être associés structurellement et fonctionnellement un tuyau 19 d'amené de la première partie d'au moins un produit à mélanger, faisant partie de moyens d'amenée (pompe par exemple).

Bien entendu, il peut être prévu non pas un seul, mais plusieurs orifices 18.

Par ailleurs, il est prévu au moins un orifice 18a de vidage de la poche 6 (et donc du récipient 2) de son contenu. Cet orifice de vidage est lui-aussi adjacent à la partie d'extrémité inférieure 12. Il peut - ou non - être commun avec l'orifice 18 et être associé à un tuyau de vidage 19a, qui peut - ou non - être le tuyau d'amené 19, faisant partie de moyens de vidage.

On décrit maintenant plus spécialement les moyens 4 de traitement, en l'espèce de mélange, dans la réalisation représentée et décrite.

Comme indiqué, les moyens 4 comportent une partie active interne 5, rigide dans son ensemble et, selon les cas, déformable ou non. Cette partie active interne 5 a une certaine hauteur H1, comptée le long de l'axe 16. Elle est disposée à l'intérieur du récipient 2. Plus précisément, la partie active interne 5 est adjacent à la partie d'extrémité inférieure 12 de la poche 6. On entend par « adjacent » le fait que la partie active interne 5 est sur (au contact de) la face interne de la partie d'extrémité inférieure 12 la face interne supérieure de la plaque rigide 17, ou proche ou au voisinage de cette partie 12 ou plaque 17. Ainsi, la partie active interne 5 est en contact avec le contenu de la poche, même en faible quantité.

Selon une réalisation possible mais non limitative, la partie active interne 5 est associée structurellement à la partie d'extrémité inférieure 12 ou la plaque rigide 17 par exemple par l'intermédiaire d'un montage à pivotement. D'autres agencements sont possibles.

Les moyens 4 comprennent généralement d'autres organes et dispositifs, tels que moteurs, commande... disposés à l'extérieur de la poche 6 et du récipient 2.

On décrit maintenant plus spécialement les moyens 7 de protection, dans la réalisation représentée (traitement consistant en un mélange).

Les moyens 7 de protection, en matière plastique, comportent une paroi latérale 20, une partie d'extrémité inférieure 21 et une partie d'extrémité supérieure 22 délimitant un espace interne 23 utile pour le logement de la partie interne active 5 des moyens 4 de traitement, de hauteur H2.

L'espace interne 23 des moyens 7 de protection est en communication avec l'espace interne 14 de la poche 6 par l'intermédiaire du passage 15 commun à la poche 6 et aux moyens 7. La partie d'extrémité inférieure 21 des moyens 7 est attenante et fixée rigidement à la partie d'extrémité supérieure 13 de la poche 6, à la périphérie du passage 15, par exemple par soudage, en 21 a.

Dans la réalisation représentée et décrite, la paroi latérale 20 est agencée pour s'étendre en direction verticale. La partie d'extrémité inférieure 21 forme une collerette s'étendant dans un plan horizontal autour et à l'extérieur de la paroi latérale 20, de manière à pouvoir venir se raccorder rigidement et de manière étanche à la partie d'extrémité supérieure 13 en forme de collerette de la poche 6.

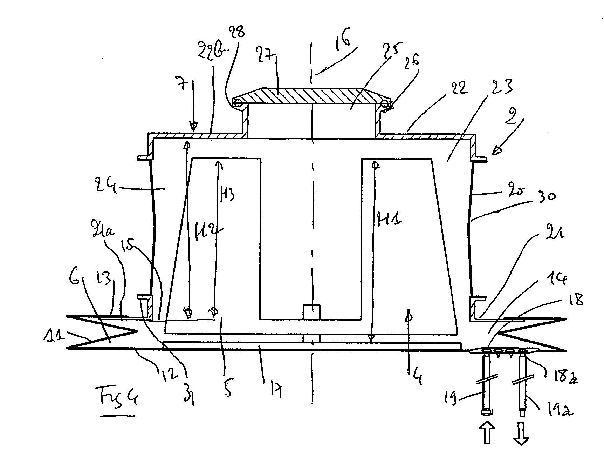

Quant à la partie d'extrémité supérieure 22, elle comporte - dans la réalisation des figures 1 à 3, un tronçon tronconique 22a, relié vers le bas à l'extrémité supérieure de la paroi latérale 20 et vers le haut à une partie formant port, sur laquelle on reviendra par la suite. Dans la réalisation des figures 4 et 5, ce tronçon 22b est plat en forme de collerette.

Selon un mode de réalisation préféré et avantageux, le dispositif 1 de traitement est tel que, lors du traitement, le conteneur 3 est fixe. La poche 6 est portée par sa partie d'extrémité inférieure 12 sur le fond 8 du conteneur 3. Lors de la mise en volume de la poche 6, résultant de ce que l'on introduit progressivement dans la poche 6 au moins un produit, la poche 6 se déploie vers le haut maintenue par l'extérieur par le conteneur 3. La partie d'extrémité supérieure 13 de la poche est donc déplacée vers le haut, tandis que la partie d'extrémité inférieure 12 reste au même niveau. Lorsque l'on vide la poche 6 de son contenu, la poche 6 se replie vers le bas, en particulier la partie d'extrémité supérieure 13 est déplacée vers le bas, la partie d'extrémité inférieure 12 restant au même niveau. C'est en référence à ce mode de réalisation préféré et avantageux et à ces déplacements que le dispositif 1 est décrit et que les expressions « vers le haut » et « vers le bas » doivent être conventionnellement comprises.

Il est entendu, cependant que les mouvements sont avant tout relatifs et que l'on peut envisager que la partie d'extrémité supérieure 13 de la poche 6 reste au même niveau, alors que la partie d'extrémité inférieure 12 est déplacée vers le haut ou vers le bas.

Référence faite au mode de réalisation préféré et avantageux décrit ci-dessus, aux déplacements qu'il induit, au sens à donner aux expressions « vers le haut » et « vers le bas », il appert que le déplacement de la partie d'extrémité supérieure 13 de la poche 6 vers le haut ou le bas entraîne un déplacement corrélatif des moyens 7 de protection. L'agencement du dispositif 1 de mélange est tel que la partie interne active 5 des moyens 4 de traitement est essentiellement disposée dans l'espace interne 23 des moyens 7 de protection lorsque la poche 6 est à l'état plié à plat.

La partie périphérique de l'espace interne 23 des moyens 7 de protection autour des moyens 4 de traitement, plus précisément de la partie interne active 5, forme une chambre de protection 24, tant de la poche 6 que des moyens 4 de traitement. La hauteur de cette chambre de protection 24, comptée le long de l'axe 16 est H3.

On entend par protection, le fait, d'abord, que la partie interne active 5 est empêchée d'interférer avec la poche 6, notamment la partie d'extrémité supérieure 13, à l'état plié et/ou lors du vidage de la poche 6, ce qui évite que le matériau constitutif de la poche 6 ne soit fragilisé, perforé ou déchiré par la partie interne active 5. Et le fait, ensuite, que la partie interne active 5 est empêchée d'interférer avec quelque chose de rigide ou en cas de choc, ce qui évite qu'elle ne soit altérée plus ou moins sérieusement, par exemple déformée ou déplacée ou encore coincée.

Les moyens 4 de mélange (traitement) et les moyens 7 de protection sont agencés pour être indépendants l'un de l'autre en ce qui concerne leurs déplacements.

Dans la réalisation représentée et décrite, les moyens 4 de traitement et les moyens 7 de protection sont dissociés structurellement les uns des autres, étant distincts les uns des autres et sans contact direct entre eux. Dans ce cas, la partie périphérique de l'espace interne 23 des moyens 7 de protection autour des moyens 4 de traitement formant la chambre de protection 24 s'étend latéralement (horizontalement) et supérieurement (verticalement et vers le haut) entre les moyens 4 de traitement et d'une part la paroi latérale 21, d'autre part la partie d'extrémité supérieure 22, des moyens 7 de protection.

D'autre part, la hauteur H3 de la chambre de protection 24 est variable. Elle peut être l'une de deux hauteurs extrêmes, maximale et minimale (et toute hauteur intermédiaire) en fonction de l'état respectivement plié à plat et déplié déployé (et tout état intermédiaire) de la poche 6. En outre, la hauteur H3 de la chambre de protection 24 s'adapte automatiquement, en fonction de l'état de la poche 6.

Les dispositions constructives décrites sont telles que dans chaque état de la poche 6, les moyens 4 de mélange (traitement) sont écartés de la paroi latérale 11 et de la partie d'extrémité supérieure 13 de la poche 6, tout en étant - et restant - adjacents à sa partie d'extrémité inférieure 12.

A l'état plié à plat de la poche 6, la partie interne active 5 des moyens 4 de traitement occupe substantiellement l'espace interne 23 des moyens 7 de protection.

A l'état déployé de la poche 6, la partie interne active 5 des moyens 4 de traitement se trouve substantiellement intégralement dans l'espace interne 14 - alors maximal - de la poche 6, la hauteur H3 de la chambre de protection 24 étant minimale et nulle.

Lors du repliement de la poche 6, consécutif à son vidage, la partie interne active 5 des moyens 4 de traitement vient se reloger dans l'espace interne 23 des moyens 7 de protection, ce qui autorise la mise en oeuvre de la partie active, notamment son déplacement, jusqu'à la fin du vidage de la poche 6.

Dans l'application particulière où le traitement est un mélange, le récipient 2 - de mélange - comporte au moins un second orifice 25 d'introduction d'une seconde partie d'au moins un produit. Ce au moins un second orifice 25 d'introduction est ménagé dans la partie d'extrémité supérieure 22 des moyens 7 de protection, étant en haut, alors que le premier orifice 18 d'introduction est en bas. Ainsi, les moyens 7 de protection forment une tête d'introduction 26 d'au moins un produit dans le récipient 2, comprenant, outre le au moins un second orifice 25, des moyens 27 d'obturation amovible du second orifice 25.

Les moyens 27 d'obturation amovible du second orifice 25 sont aptes à assurer sa fermeture et son ouverture.

Dans la réalisation représentée sur les figures 1 et 4, les moyens 27 d'obturation amovible du second orifice 25 comprennent un bouchon maintenu en place sur une collerette 28 prévue sur le bord libre de la partie d'extrémité supérieure 22 formant un port ayant une forme générale de cheminée autour de l'orifice 25, grâce à un système de maintien approprié, amovible.

Dans la réalisation représentée sur la figure 2, les moyens 27 d'obturation amovible du second orifice 25 comprennent une membrane fixée par collage ou soudage sur le bord libre de la partie d'extrémité supérieure 22 en forme générale de cheminée. Cette membrane peut être enlevée par pelage, notamment grâce à une languette de tirage 29.

Bien entendu, ces moyens 27 d'obturation amovible ne sont pas exclusifs d'autres réalisations.

Par ailleurs, la tête d'introduction 26 peut, dans une réalisation non représentée, comprendre des moyens d'association amovible du récipient 2 à un dispositif de transfert aseptique, du type de ceux comportant une poche de transfert aseptique.

Dans ces conditions, le dispositif 1 de mélange permet, selon les réalisations, un ajout de produit de façon aseptique ou non aseptique.

Les moyens 7 de protection assurent en combinaison non seulement la fonction de protection de la poche 6 et des moyens 4 de traitement, décrite précédemment, mais également une fonction de guidage de la poche 6, lorsque, l'orifice 18 étant ouvert, on introduit progressivement dans le récipient 2 le au moins un produit (flèche R sur les figures 3 et 5), alors que simultanément la poche 6 se déploie vers le haut, ainsi que lors du vidage de la poche 6.

On décrit maintenant une première réalisation des moyens 7 de protection, en référence aux figures 1 à 3. Dans cette première réalisation, les moyens 7 de protection - et notamment la paroi latérale 20 - sont rigides et indéformables. Les moyens 7 de protection ont alors une forme générale correspondant à un large prisme terminé par une collerette en partie basse, une cheminée cylindrique de plus petit diamètre en partie haute et, entre les deux, une partie tronconique.

On décrit maintenant une seconde réalisation des moyens 7 de protection, en référence aux figures 4 et 5. Dans cette seconde réalisation, les moyens 7 de protection - et notamment la paroi latérale 20 - sont déformables en hauteur.

Dans cette seconde réalisation, la paroi latérale 20 des moyens 7 de protection comprend au moins un tronçon 30 d'une part déformable entre deux états extrêmes, respectivement plié à plat et déployé verticalement, d'autre part apte à être déformé pour passer d'un état à l'autre. Le cas échéant, c'est l'ensemble de la paroi latérale 20 qui est déformable.

Outre une déformabilité appropriée du tronçon déformable 30, il est prévu des moyens adaptés, aptes à maintenir le tronçon déformable 30 déployé, lorsque cela est souhaité, notamment au moins à l'état extrême plié à plat de la poche, et en l'absence de sollicitation.

Dans une réalisation, de tels moyens aptes à maintenir le tronçon déformable 30 déployé sont intégrés au tronçon déformable 30 et à la paroi latérale 20 qui est du type compressible. On entend par là que le tronçon déformable 30 comporte des nervures périphériques aptes, en l'absence de sollicitation extérieure, à maintenir le tronçon déformable déployé mais pouvant, par suite d'une sollicitation extérieure de compression (appui vers le bas) se déformer de manière que le tronçon déformable 30 se plie en accordéon.

Dans une autre réalisation, dans laquelle le tronçon déformable 30 est souple, les moyens aptes à maintenir le tronçon déformable 30 déployé sont distincts de la paroi latérale 20. Ils peuvent consister en un support extérieur amovible, susceptible d'être lui-même porté par le conteneur 3 vers son ouverture 10.

Par ailleurs, étant destiné à être disposé dans le conteneur 3, le récipient 2 comporte avantageusement des moyens 31 aptes à participer à son maintien dans le conteneur 3, la poche 6 contenant la quantité souhaitée du au moins un produit.

Selon une réalisation, de tels moyens 31 sont intégrés ou associés aux moyens 7 de protection. Ils peuvent consister, par exemple, en une collerette externe ménagée sur la face externe de la paroi latérale 20 des moyens 7 de protection vers la partie d'extrémité inférieure 21. Une telle collerette 31 peut alors coopérer avec une partie conjuguée prévue à cet effet dans le conteneur 3, telle que le retour en collerette 10a. Selon une réalisation, cette partie conjuguée comprend deux traverses amovibles, montées sur le conteneur 3, portées par et fixées de façon amovible au conteneur 3 vers son ouverture supérieure et disposées sous la collerette 31 une fois que la poche 6 a été emplie.

Un récipient 6 de traitement (mélange) tel que décrit précédemment, peut se trouver dans une situation de stockage avant emploi. La poche 6 qui renferme la partie interne active 5 des moyens 4 de traitement est alors à l'état plié. Dans cet état, la poche 6 a l'encombrement extérieur minimal et peut être aisément stockée et manipulée. Il est entendu que dans cette situation, la poche 6 est fermée, les orifices 18, 25 étant obturés.

Pour mettre en oeuvre le dispositif 1 de traitement (mélange), on dispose d'un tel récipient 2 et d'un conteneur 3, les deux étant constitutifs du dispositif 1. On place la poche 6 du récipient à l'état plié à plat dans le conteneur 3, sur le fond 8. Comme indiqué précédemment, la partie interne active 5 des moyens 4 de traitement occupe alors substantiellement l'espace interne 23 des moyens 7 de protection, en étant ainsi écartée de la paroi latérale 11 et de la partie d'extrémité supérieure 13 de la poche 6. La poche 6 et les moyens 4 de traitement sont ainsi protégés (figures 1, 2 et 4). La chambre de protection 24 est alors maximale.

Le premier orifice 18 d'introduction dans la poche 6 d'une première partie d'au moins un produit à mélanger étant ouvert, on introduit progressivement dans le récipient de mélange 2 une première partie d'au moins un produit.

Simultanément on laisse la poche 6 se déployer vers le haut et être mise en volume par le conteneur 3.

L'espace interne 14 de la poche 6 voit son volume augmenter progressivement, recevant le au moins un produit.

On poursuit cette étape jusqu'à avoir introduit dans le récipient 2 la quantité souhaitée du au moins un produit.

Durant cette étape, on laisse les moyens 7 de protection se déplacer vers le haut, étant entraînés par le déplacement de la partie d'extrémité supérieure 13 de la poche 6.

Par ailleurs, durant cette étape, et au fur et à mesure que les moyens 7 de protection se déplacent vers le haut, on laisse la hauteur de la chambre de protection 24 diminuer à partir de la hauteur extrême maximale précédente et s'adapter automatiquement en fonction de l'état de la poche 6 jusqu'à ce que la poche 6 soit dans l'état déplié et déployé - ou tout état intermédiaire - en fonction de la quantité de produit introduite dans la poche 6 (figures 3 et 5). La partie interne active 5 des moyens 4 de traitement est alors entièrement libérée de la chambre de protection 24 pour se trouver intégralement dans l'espace interne 14 de la poche 6.

Dans le cas de la seconde réalisation des moyens 7 de protection, le tronçon déformable 30 est à l'état déployé au moins au début du mouvement vers le haut des moyens 7 de protection. Une fois la poche 6 dans l'état déplié et déployé, le tronçon déformable 30 peut être comprimé. Cette disposition constitutive permet de limiter l'encombrement vers le haut du dispositif 1 et de loger celui-ci intégralement ou quasi intégralement dans le conteneur 3. Le cas échéant, on comprime le tronçon déformable 30 au fur et à mesure du mouvement vers le haut des moyens 7 de protection.

Dans tous les états de la poche, la partie interne active 5 des moyens 4 de traitement est écartée de la paroi latérale 11 et de la partie d'extrémité supérieure 13 de la poche 6, tout en étant - et restant - adjacents à sa partie d'extrémité inférieure 12. La poche 6 et les moyens 4 de traitement restent ainsi toujours protégés, par une sorte d'écran de protection permanent et modulaire entre la poche 6 et la partie interne active 5.

Le procédé comporte également une étape dans laquelle on met en oeuvre les moyens 4 de traitement du au moins un produit se trouvant dans le récipient 2.

Dans le cas où le traitement est un mélange, on introduit dans le récipient 2 une seconde partie d'au moins un produit par le second orifice d'introduction 25 ouvert à cette fin (flèche R, figures 3 et 5). Par exemple, on introduit un produit à l'état solide, alors que le récipient 2 contient un produit à l'état liquide.

Selon les réalisations, l'introduction de la seconde partie d'au moins un produit par le second orifice d'introduction 25 débute dès qu'une quantité suffisante de la première partie du produit se trouve dans la poche 6, ou lorsque la totalité de la première partie du produit a été introduite dans la poche 6.

Selon les réalisations, on commence la mise en oeuvre des moyens 4 de mélange du au moins un produit se trouvant dans le récipient 2 avant, au moment ou après avoir introduit dans le récipient 2 la quantité souhaitée du au moins un produit. Et on met en oeuvre les moyens 4 de mélange alors que le au moins un second orifice d'introduction 25 est ouvert ou fermé.

Comme indiqué précédemment, il est entendu que les mouvements doivent être considérés comme des mouvements relatifs.

Lors du vidage du récipient 2, on ouvre l'orifice de vidage 18a prévu à cet effet. Contrairement à la pratique de l'état de la technique, il est possible de vider complètement la poche 6, sans risque de déchirement, grâce aux moyens 7 de protection formant écran. En outre, il est possible de poursuivre le mélange jusqu'au vidage complet, la partie active 5 étant protégée, comme il a été décrit.

Le procédé de mélange a donc également pour caractéristique optionnelle que l'on met en oeuvre les moyens 4 de mélange jusqu'à vidage complet ou substantiellement complet de la poche 6.

## Revendications

1. Récipient (2) de traitement d'au moins un produit biopharmaceutique, comprenant :
■ une poche (6) ayant une paroi latérale (11) souple, une partie d'extrémité inférieure (12) et une partie d'extrémité supérieure (13) délimitant un espace interne (14) destiné à recevoir le au moins un produit, pouvant se trouver dans deux états extrêmes, plié à plat et déployé, et dans tout état intermédiaire, et être déformée pour passer d'un état à un autre ;
■ des moyens (4) de traitement du au moins un produit contenu dans l'espace (14) de réception de la poche (6), comportant une partie interne active (5) d'une certaine hauteur disposée à l'intérieur du récipient (2);
■ des moyens de protection (7) des moyens (4) de traitement, comportant une paroi latérale (20), une partie d'extrémité inférieure (21) et une partie d'extrémité supérieure (22) délimitant un espace interne (23) en communication avec l'espace interne (14) de la poche (6), dont la partie d'extrémité inférieure (21) est attenante et fixée rigidement à la partie d'extrémité supérieure (13) de la poche (6), le déplacement de celle-ci vers le haut ou le bas entraînant un déplacement des moyens (7) de protection, la partie interne active (5) des moyens (4) de traitement étant essentiellement disposée dans l'espace interne (23) des moyens (7) de protection lorsque la poche (6) est à l'état plié à plat, la partie périphérique de l'espace interne (23) des moyens (7) de protection autour des moyens (4) de traitement formant une chambre de protection (24) de la poche (6) et des moyens (4) de traitement ;
■ au moins un orifice (18) d'introduction dans le récipient (2) du au moins un produit
et/ou de vidage du récipient (2) de son contenu ;
**caractérisé en ce que** :
■ les moyens (4) de traitement sont au moins pour une partie interne active (5) - organe de mélange, capteur, tube, organe d'aération ou autre - disposés à l'intérieur de la poche (6) en étant adjacents à sa partie d'extrémité inférieure (12) ;
■ les moyens (4) de traitement et les moyens (7) de protection sont agencés pour être indépendants l'un de l'autre en ce qui concerne leurs déplacements ;
■ la hauteur de la chambre de protection (24), variable, peut être l'une de deux hauteurs extrêmes, maximale et minimale, et toute hauteur intermédiaire, et s'adapter automatiquement, en fonction de l'état respectivement plié à plat, déplié et déployé, et tout état intermédiaire, de la poche (6) ;
■ de manière que dans chaque état de la poche (6), les moyens (4) de traitement sont écartés de la paroi latérale (11) et de la partie d'extrémité supérieure (12) de la poche (6), tout en étant adjacents à sa partie d'extrémité inférieure (13).

2. Récipient (2) de traitement selon la revendication 1, **caractérisé en ce que** les moyens (7) de protection sont rigides et indéformables.

3. Récipient (2) de traitement selon la revendication 1, **caractérisé en ce que** les moyens (7) de protection sont déformables en hauteur, la paroi latéral (20) comprenant au moins un tronçon déformable (30) entre deux états extrêmes, respectivement plié et déployé, apte à être déformé pour passer d'un état à l'autre ; le récipient (2) comprenant en outre des moyens aptes à maintenir le tronçon déformable (30) déployé, au moins à l'état extrême plié à plat de la poche (6).

4. Récipient (2) de traitement selon la revendication 3, **caractérisé en ce que** les moyens aptes à maintenir le tronçon déformable (30) déployé, au moins à l'état extrêmes, plié à plat de la poche (6) sont intégrés à la paroi latérale (20) des moyens (7) de protection ou distincts d'elle.

5. Récipient (2) de traitement selon l'une quelconque des revendications 1 à 4, **caractérisé en ce que** les moyens (7) de protection sont agencés pour former une tête d'introduction (26) d'au moins un produit dans le récipient (2).

6. Récipient (2) de traitement selon la revendication 5, **caractérisé en ce que** les moyens (7) de protection formant une tête d'introduction (26) d'au moins un produit dans le récipient (2) comprennent au moins un orifice d'introduction (25) du dit au moins produit ménagé dans la partie d'extrémité supérieure (22) des moyens (7) de protection et des moyens d'obturation amovible du dit orifice aptes à assurer sa fermeture et son ouverture.

7. Récipient (2) de traitement selon la revendication 6, **caractérisé en ce que** les moyens d'obturation amovible (27) du dit orifice d'introduction (25) du dit au moins un produit sont un bouchon ou une membrane.

8. Récipient (2) de traitement selon l'une quelconque des revendications 5 à 7, **caractérisé en ce que** la tête d'introduction (26) d'au moins un produit dans le récipient (2) comprend des moyens d'association amovible du récipient (2) à un dispositif de transfert aseptique.

9. Récipient (2) de traitement selon l'une quelconque des revendications 1 à 8, **caractérisé par** au moins un orifice (18) d'introduction dans le récipient (2) d'au moins un produit et/ou de vidage du récipient (2) de son contenu ménagé sur la poche (6).

10. Récipient (2) de traitement selon la revendication 9, **caractérisé** un orifice (18) d'introduction dans le récipient (2) d'au moins un produit et/ou de vidage du récipient (2) de son contenu ménagé dans la partie d'extrémité inférieure (12) de la poche (6).

11. Récipient (2) de traitement selon l'une quelconque des revendications 1 à 10, **caractérisé par** une poche (6) garnie à l'intérieur d'une plaque rigide (17) sur sa partie d'extrémité inférieure (12).

12. Récipient (2) de traitement selon l'une quelconque des revendications 1 à 11, **caractérisé par** des moyens (7) de protection des moyens (4) de traitement, assurant en combinaison la fonction de protection de la poche (6) et des moyens (4) de traitement et une fonction de guidage de la poche (6) lorsque, l'orifice (18) étant ouvert, on introduit progressivement dans le récipient (2) le au moins un produit, alors que simultanément la poche (6) se déploie vers le haut.

13. Récipient (2) de traitement selon l'une quelconque des revendications 1 à 12, **caractérisé par** des moyens (4) de traitement et des moyens (7) de protection dissociés structurellement les uns des autres étant distincts les uns des autres et sans contact direct entre eux, la partie périphérique de l'espace interne (23) des moyens (7) de protection autour des moyens (4) de traitement formant une chambre de protection (24) s'étendant latéralement et supérieurement entre les moyens (4) de traitement et la paroi latérale (20) et la partie d'extrémité supérieure (22) des moyens (7) de protection.

14. Récipient (2) de traitement selon l'une quelconque des revendications 1 à 13, **caractérisé par** des moyens (4) de traitement ayant une partie active (5) à l'intérieur de la poche (6) qui, à l'état plié à plat de la poche (6), occupe substantiellement l'espace interne (23) des moyens (7) de protection.

15. Récipient (2) de traitement selon l'une quelconque des revendications 1 à 14, **caractérisé par** des moyens (4) de traitement ayant une partie active (5) à l'intérieur de la poche (6) qui, à l'état déployé de la poche (6), se trouve substantiellement intégralement dans l'espace interne (14) de la poche (6), la hauteur minimale de la chambre de protection (24) étant nulle.

16. Récipient (2) de traitement selon l'une quelconque des revendications 1 à 15, **caractérisé par** des moyens (4) de traitement ayant une partie active (5) à l'intérieur de la poche (6) portée, le cas échéant avec possibilité de déplacement relatif, par la face intérieure de la partie d'extrémité inférieure (12) de la poche (6) ou par une plaque rigide (17) garnissant la partie d'extrémité inférieure (12) de la poche (6) à l'intérieur.

17. Récipient (2) de traitement selon l'une quelconque des revendications 1 à 16, **caractérisé par le fait qu'**il comporte des moyens (31) aptes à participer à son maintien dans un conteneur (3), la poche (6) contenant la quantité souhaitée du au moins un produit.

18. Récipient (2) de traitement selon la revendication 17, **caractérisé par le fait que** les moyens (31) aptes à participer à son maintien dans un conteneur (3), la poche (6) contenant la quantité souhaitée du au moins un produit, sont intégrés ou associés aux moyens (7) de protection.

19. Récipient (2) de mélange **caractérisé par** la fait qu'il est constitué par un récipient (2) selon l'une quelconque des revendications 1 à 18, dans lequel les moyens (4) de traitement sont des moyens de mélange, le récipient (2) comportant au moins un premier orifice (18) d'introduction dans le récipient (2) d'une première partie d'au moins un produit ménagé sur la poche (6), notamment adjacent à sa partie d'extrémité inférieure (12) et au moins un second orifice (25) d'introduction d'une seconde partie d'au moins un produit ménagé dans la partie d'extrémité supérieure (22) des moyens (7) de protection.

20. Dispositif (1) de traitement d'au moins un produit biopharmaceutique, comprenant un récipient (2) selon l'une quelconque des revendications 17, 18 et 19 en ce qu'elle dépend des revendications 17 et 18, et un conteneur rigide (3) apte à recevoir le récipient (2) dont la poche (6) est à l'état plié à plat, à l'état déployé, ou dans un état intermédiaire, à permettre à la poche (6) d'être déformée pour passer d'un état à un autre, et à guider la poche (6), notamment sa paroi latérale souple (11), afin que la poche (6) soit mise en volume, avec un volume complémentaire de celui du conteneur (3), lorsque l'on introduit dans le récipient (2) le au moins un produit.

21. Procédé de mise en oeuvre d'un dispositif (1) de traitement selon la revendication 20, dans lequel :
■ on dispose d'un récipient (2) et d'un conteneur (3) constitutifs du dispositif (1) de traitement et on place la poche (6) du récipient à l'état plié à plat dans le conteneur (3), sur le fond (8);
■ un orifice (18) d'introduction de produit du récipient (2) étant ouvert, on introduit progressivement dans le récipient (2) le au moins un produit et simultanément on laisse la poche (6) se déployer vers le haut et être mise en volume par le conteneur (3), l'espace interne (14) de la poche (6), de volume croissant, recevant le au moins un produit ; on poursuit cette étape jusqu'à avoir introduit dans le récipient (2) la quantité souhaitée du au moins un produit ;
■ durant cette étape, on laisse les moyens (7) de protection se déplacer vers le haut étant entraînés par le déplacement de la partie d'extrémité supérieure (13) de la poche (6);
■ durant cette étape, on laisse la hauteur de la chambre de protection (24) diminuer à partir de la hauteur extrême maximale et s'adapter automatiquement en fonction de l'état de la poche (6) jusqu'à ce que la poche (6) soit dans l'état déplié et déployé ou tout état intermédiaire, en fonction de la quantité de produit introduite dans le récipient (2), de manière que dans tous les états de la poche (6), les moyens (4) de traitement soient écartés de la paroi latérale (11) et de la partie d'extrémité supérieure (13) de la poche (6), tout en étant adjacents à sa partie d'extrémité inférieure (12) ;
■ on met en oeuvre les moyens (4) de traitement du au moins un produit se trouvant dans le récipient (2).

22. Procédé selon la revendication 21, **caractérisé par** le fait: on commence la mise en oeuvre des moyens (4) de traitement du au moins un produit se trouvant dans le récipient (2) avant, au moment ou après avoir introduit dans le récipient (2) la quantité souhaitée du au moins un produit.

23. Procédé de mélange, dans lequel on met en oeuvre le procédé selon l'une quelconque des revendications 21 et 22, **caractérisé par le fait: que** :
■ on dispose d'un récipient (2) de mélange selon la revendication 19 dont le au moins un second orifice (25) est obturé ;
■ on place la poche (6) du récipient (2) à l'état plié à plat dans le conteneur (3), sur le fond (8) ;
■ on introduit dans le récipient (2) de mélange une première partie d'au moins un produit par au moins un premier orifice (18) d'introduction ouvert à cette fin ;
■ et on introduit dans le récipient (2) une seconde partie d'au moins un produit par le second orifice d'introduction (25) ouvert à cette fin.

24. Procédé de mélange selon la revendication 23, **caractérisé par le fait que** l'on met en oeuvre les moyens (4) de mélange alors que le au moins un second orifice d'introduction (25) est ouvert ou fermé.

25. Procédé de mélange selon l'une quelconque des revendications 23 et 24, **caractérisé par le fait que** l'on met en oeuvre les moyens (4) de mélange jusqu'à vidage complet ou substantiellement complet de la poche (6).

## Claims

1. A vessel (2) for processing at least one biopharmaceutical product, including:
■ a bag (6) having a flexible side wall (11), a lower end part (12) and an upper end part (13) defining an internal space (14) intended to receive the at least one product, which can be in two extreme, flat folded and expanded conditions, and in any intermediate condition, and be deformed to change from one condition to another condition;
■ means (4) for processing the at least one product filling the bag (6) receiving space (14), including an internal active part (5) having a certain height, positioned inside the vessel (2);
■ means (7) for protecting the processing means (4) including a side wall (20), a lower end part (21) and an upper end part (22) defining an internal space (23) communicating with the internal space (14) of the bag (6), the lower end part (21) of which is contiguous with and rigidly fixed to the upper end part (13) of the bag (6); the motion thereof upwards or downwards causing a motion of the protection means (7), with the internal active part (5) of the processing means (4) being substantially positioned in the internal space (23) of the protection means (7) when the bag (6) is in a flat folded condition, with the peripheral part of the internal space (23) of the protection means (7) around the processing means (4) forming a chamber (24) protecting the bag (6) and the processing means (4);
■ at least one opening (18) for introducing into the vessel (2) at least one product and / or for emptying the vessel (2) of its content;
**characterized in that**:
■ the processing means (4) are, at least for an internal active part (5) -mixing member, detector, tube, venting member, or any other member- positioned inside the bag (6), while being adjacent to the lower end part (12) thereof;
■ the processing means (4) and the protection means (7) are so arranged as to be independent from one another, as far as their motions are concerned;
■ the variable height of the protection chamber (24) can be one of two extreme, maximum and minimum heights, and any other intermediate height, and automatically fit, as a function of the respectively flat, folded, unfolded and expanded condition and any intermediate condition, of the bag (6);
■ so that in each condition of the bag (6), the processing means (4) are kept away from the side wall (11) and from the upper end part (12) of the bag (6), while being adjacent to the lower end part (13) thereof.

2. A processing vessel (2) according to claim 1, **characterized in that** the protection means (7) are rigid and non deformable.

3. A processing vessel (2) according to claim 1, **characterized in that** the protection means (7) can be deformed in height, with the side wall (20) including at least one segment (30) deformable between two extreme, respectively folded and expanded conditions, able to be deformed to change from one condition to another condition ; with the vessel (2) further including means able to keep expanded the deformable segment (30), at least in the extreme flat folded condition of the bag (6).

4. A processing vessel (2) according to claim 3, **characterized in that** the means able to keep expanded the deformable segment (30), at least in the extreme, flat folded condition of the bag (6), are integrated in the side wall (20) of the protection means (7) or separated therefrom.

5. A processing vessel (2) according to any one of claims 1 to 4, **characterized in that** the protection means (7) are so arranged as to form a head for introducing (26) at least one product into the vessel (2).

6. A processing vessel (2) according to claim 5, **characterized in that** the protection means (7) forming a head for introducing (26) at least one product into the vessel (2) include at least one opening (25) for introducing said at least one product arranged in the upper end part (22) of the protection means (7) and means for removably closing said opening able to provide the opening and closing thereof.

7. A processing vessel (2) according to claim 6, **characterized in that** the means for removably closing (27) said opening (25) for introducing said at least one product are a cork or a membrane.

8. A processing vessel (2) according to any one of claims 5 to 7, **characterized in that** the head (26) for introducing the at least one product into the vessel (2) includes means for removably associating the vessel (2) with an aseptic transfer device.

9. A processing vessel (2) according to any one of claims 1 to 8, **characterized by** at least one opening (18) for introducing into the vessel (2) at least one product and / or for emptying the vessel (2) of its content, arranged on the bag (6).

10. A processing vessel (2) according to claim 9, **characterized by** one opening (18) for introducing into the vessel (2) at least one product and / or for emptying the vessel (2) of its content, arranged in the lower end part (12) of the bag (6).

11. A processing vessel (2) according to any one of claims 1 to 10, **characterized by** a bag (6) provided inside a rigid plate (17), on the lower end part (12) thereof.

12. A processing vessel (2) according to any one of claims 1 to 11, **characterized by** means (7) for protecting the processing means (4), providing, in combination, the function of protecting the bag (6) and processing means (4) and a function of guiding the bag (6) when, with the opening (18) being open, the at least one product is progressively introduced into the vessel (2), whereas the bag (6) simultaneously expands upwards.

13. A processing vessel (2) according to any one of claims 1 to 12, **characterized by** processing means (4) and protection means (7) structurally apart from each other, and being distinct from each other and without any direct contact together, the peripheral part of the internal space (23) of the protection means (7) about the processing means (4) forming a protection chamber (24) extending laterally and upwards between the processing means (4) and the side wall (20) and the upper end part (22) of the protection means (7).

14. A processing vessel (2) according to any one of claims 1 to 13, **characterized by** processing means (4) having an active part (5) inside the bag (6) which, when in a flat, folded condition of the bag (6) substantially fills the internal space (23) of the protection means (7).

15. A processing vessel (2) according to any one of claims 1 to 14, **characterized by** processing means (4) having an active part (5) inside the bag (6) which, in the expanded condition of the bag (6) is substantially completely in the internal space (14) of the bag (6), with the minimum height of the protection chamber (24) being null.

16. A processing vessel (2) according to any one of claims 1 to 15, **characterized by** processing means (4) having an active part (5) inside the bag (6) supported, if need be, with a relative motion being possible, by the internal face of the lower end part (12) of the bag (6) or by a rigid plate (17) filling the inside of the lower end part (12) of the bag (6).

17. A processing vessel (2) according to any one of claims 1 to 16, **characterized in that** it includes means (31) able to participate in the keeping thereof in a container (3), with the bag (6) containing the desired quantity of the at least one product.

18. A processing vessel (2) according to claim 17, **characterized in that** the means (31) able to participate in the keeping thereof in a container (3), with the bag (6) containing the desired quantity of the at least one product, are integrated in or associated with the protection means (7).

19. A mixing vessel (2) **characterized in that** it is composed of a vessel (2) according to any one of claims 1 to 18, wherein the processing means (4) are mixing means, with the vessel (2) including at least one opening (18) for the introduction in the vessel (2) of a first part of at least one product arranged in the bag (6), more particularly adjacent to the lower end part (12) thereof and at least one second opening (25) for the introduction of a second part of at least one product arranged in the upper end part (22) of the protection means (7).

20. A device (1) for processing at least one biopharmaceutical product including a vessel (2) according to any one of claims 17, 18 and 19 when depending on claims 17 and 18, and a rigid container (3) able to support the vessel (2) the bag (6) of which is in a flat, folded, expanded or intermediate condition, for enabling the bag (6) to be deformed to change from one condition to another, and for guiding the bag (6), more particularly the flexible side wall (11) thereof, so that the bag (6) can take volume, with a volume complementary to that of the container (3), when the at least one product is introduced into the vessel (2).

21. A method for implementing a processing device (1) according to claim 20, wherein:
■ a vessel (2) and a container (3) composing the processing device (1) are provided and the bag (6) of the vessel is placed in a flat, folded condition in the container (3), on the bottom (8) thereof,
■ as a product introduction opening (18) of the vessel (2) is open, the at least one product is progressively introduced into the vessel (2) simultaneously letting the bag (6) expand upwards and take volume in the container (3), with the internal space (14) of the bag (6), having an increasing volume, receiving the at least one product; this step is continued until the desired quantity of the at least one product is introduced into the vessel (2);
■ during this step, the protection means (7) are left to move upwards, being moved by the motion of the upper end part (13) of the bag (6);
■ during this step, the height of the protection chamber (24) is left to decrease from the maximum extreme height and to automatically fit according to the condition of the bag (6), until the bag (6) is in a folded or expanded condition, or any other intermediate condition, depending on the quantity of product introduced into the vessel (2), so that, in all the conditions of the bag (6), the processing means (4) are separated from the side wall (11) and the upper end part (13) of the bag (6), while remaining adjacent to the lower end part (12) thereof;
■ the means (4) for processing the at least one product present in the vessel (2) is implemented.

22. A method according to claim 21, **characterized in that**: the implementation of the means (4) for processing the at least one product present in the vessel (2) is started before, as or after the desired quantity of the at least one product is introduced into the vessel (2).

23. A mixing method, wherein the method according to any one of claims 21 and 22 is implemented, **characterized in that**:
■ a mixing vessel (2) according to claim 19 is provided, the at least one second opening (25) of which is closed;
■ the bag (6) of the vessel (2) is placed in a flat, folded condition in the container (3), on the bottom (8) thereof,
■ a first part of at least one product is introduced into the mixing vessel (2) through a first introduction opening (18) opened therefor,
■ and a second part of at least one product is introduced into the vessel (2) through the second introduction opening (25) opened therefor.

24. A mixing method according to claim 23, **characterized in that** the mixing means (4) are implemented whereas the at least second introduction opening (25) is open or closed.

25. A mixing method according to any one of claims 23 and 24, **characterized in that** the mixing means (4) is implemented until the complete or substantially complete emptying of the bag (6).

## Patentansprüche

1. Verarbeitungsbehälter (2) wenigstens eines biopharmazeutischen Produkts, umfassend einen Beutel (6) mit einer elastischen lateralen Wand (11), einem unteren Endteil (12) und einem oberen Endteil (13), die einen inneren Raum (14) begrenzen, der zur Aufnahme wenigstens eines Produkts bestimmt ist, der sich in zwei extremen Zuständen - flach gefaltet und ausgestreckt und in jedem Zwischenzustand befinden und verformt werden kann, um von einem Zustand zum anderen überzugehen;
Verarbeitungsmittel (4) des wenigstens einen in dem Aufnahmeraum (14) des Beutels (6) enthaltenen Produkts, der einen aktiven internen Teil (5) einer bestimmten, im Innern des Behälters (2) angeordneten Höhe umfasst;
Schutzmittel (7) der Verarbeitungsmittel (4), die eine laterale Wand (20), einen unteren Endteil (21) und einen oberen Endteil (22) umfassen, die einen inneren Raum (23) in Verbindung mit dem inneren Raum (14) des Beutels (6) begrenzen, dessen unterer Endteil (21) am oberen Endteil (13) des Beutels (6) anliegend und steif befestigt ist, wobei die Verschiebung desselben nach oben oder unten eine Verschiebung der Schutzmittel (7) nach sich zieht, wobei der aktive interne Teil (5) der Bearbeitungsmittel (4) im Wesentlichen im internen Raum (23) der Schutzmittel (7) angeordnet ist, wenn der Beutel (6) sich im flach gefalteten Zustand befindet, wobei der peripherische Teil des internen Raums (23) der Schutzmittel (7) um Verarbeitungsmittel (4) eine Schutzkammer (24) des Beutels (6) und der Verarbeitungsmittel (4) bildet;
wenigstens eine Einführungsöffnung (18) in dem Behälter (2) des wenigstens einen Produkts und / oder Leerungsöffnung seines Inhaltes aus dem Behälter (2):
**dadurch gekennzeichnet, dass**:
die Verarbeitungsmittel (4) wenigstens zu einem aktiven internen Teil (5) - Mischorgan, Sensor, Röhre, Belüftungsorgan oder Sonstiges - im Innern des Beutels (6) angeordnet und dabei an seinem unteren Endteil (12) anliegend sind;
die Verarbeitungsmittel (4) und die Schutzmittel (7) angeordnet sind, um voneinander unabhängig zu sein, was ihre Verschiebungen anbelangt;
die variable Höhe der Schutzkammer (24) eine der zwei extremen Höhen - maximal und minimal - und jede Zwischenhöhe sein kann und sich automatisch in Abhängigkeit von dem jeweiligen flach gefalteten, entfalteten und ausgestreckten Zustand und jedem Zwischenzustand des Beutels (6) anpassen.
derart, dass die Verarbeitungsmittel (4) in jedem Zustand des Beutels (6) von der lateralen Wand (11) und dem oberen Endteil (12) des Beutels (6) beabstandet und dabei gleichzeitig an seinem unteren Endteil (13) anliegend sind.

2. Verarbeitungsbehälter (2) gemäß Anspruch 1, **dadurch gekennzeichnet, dass** die Schutzmittel (7) steif und unverformbar sind.

3. Verarbeitungsbehälter (2) gemäß Anspruch 1, **dadurch gekennzeichnet, dass** die Schutzmittel (7) in der Höhe verformbar sind, wobei die laterale Wand (20) wenigstens einen zwischen zwei extremen, jeweils gefalteten und ausgestreckten Zuständen verformbaren Abschnitt (30) umfasst, der geeignet ist, verformt zu werden, um von einem Zustand zum anderen überzugehen; wobei der Behälter (2) darüber hinaus Mittel umfasst, die geeignet sind, den ausgestreckten verformbaren Abschnitt (30) wenigstens im flach gefalteten Zustand des Beutels (6) festzuhalten.

4. Verarbeitungsbehälter (2) gemäß Anspruch 3, **dadurch gekennzeichnet, dass** die zum Festhalten des ausgestreckten verformbaren Abschnitts (30) wenigstens im flach gefalteten Endzustand des Beutels (6) geeigneten Mittel in die laterale Wand (20) der Schutzmittel (7) integriert oder von ihr unterschiedlich sind.

5. Verarbeitungsbehälter (2) gemäß Anspruch 1 bis 4, **dadurch gekennzeichnet, dass** die Schutzmittel (7) angeordnet sind, um einen Einführkopf (26) wenigstens eines Produkts in den Behälter (2) zu bilden.

6. Verarbeitungsbehälter (2) gemäß Anspruch 5, **dadurch gekennzeichnet, dass** die einen Einführkopf (26) wenigstens eines Produkts in den Behälter (2) bildenden Schutzmittel (7) wenigstens eine Einführöffnung (25) des genannten wenigstens einen Produkts umfassen, die im oberen Endteil (22) der Schutzmittel (7) ausgespart ist, und abnehmbare Verschlussmittel der genannten Öffnung, die geeignet sind, seinen Verschluss und seine Öffnung zu gewährleisten.

7. Verarbeitungsbehälter (2) gemäß Anspruch 6, **dadurch gekennzeichnet, dass** die abnehmbaren Verschlussmittel (27) der genannten Einführöffnung (25) des genannten wenigstens einen Produkts ein Stopfen oder eine Membran sind.

8. Verarbeitungsbehälter (2) gemäß Anspruch 5 bis 7, **dadurch gekennzeichnet, dass** der Einführkopf (26) wenigstens eines Produkts in dem Behälter (2) abnehmbare Zuordnungsmittel des Behälters (2) zu einer aseptischen Transfervorrichtung umfasst.

9. Verarbeitungsbehälter (2) gemäß Anspruch 1 bis 8, **dadurch gekennzeichnet, dass** wenigstens eine Einführöffnung (18) in den Behälter (2) wenigstens eines Produkts und / oder Leerungsöffnung seines Inhaltes aus dem Behälter (2) auf dem Beutel ausgespart ist.

10. Verarbeitungsbehälter (2) gemäß Anspruch 9, **gekennzeichnet**, dass eine Einführöffnung (18) in den Behälter (2) wenigstens eines Produkts und / oder Leerungsöffnung seines Inhaltes aus dem Behälter (2) im unteren Endteil (12) des Beutels (6) ausgespart ist.

11. Verarbeitungsbehälter (2) gemäß Anspruch 1 bis 10, **gekennzeichnet durch** einen Beutel (6), der im Innern einer festen Platte (17) auf seinem unteren Endteil (12) bestück ist.

12. Verarbeitungsbehälter (2) gemäß Anspruch 1 bis 11, **gekennzeichnet durch** Schutzmittel (7) der Verarbeitungsmittel (4), die in Kombination die Schutzfunktion des Beutels (6) und der Verarbeitungsmittel (4) und eine Führungsfunktion des Beutels (6) gewährleisten, wenn die Öffnung (18) offen ist, wird progressiv in den Behälter (2) das wenigstens eine Produkt eingeführt, während der Beutel (6) sich gleichzeitig nach oben ausstreckt.

13. Verarbeitungsbehälter (2) gemäß Anspruch 1 bis 12, **gekennzeichnet durch** Verarbeitungsmittel (4) und Schutzmittel (7), die strukturell voneinander getrennt, voneinander unterschiedlich und untereinander ohne direkten Kontakt sind, wobei der peripherische Teil des inneren Raums (23) der Schutzmittel (7) um Verarbeitungsmittel (4) eine Schutzkammer (24) bilden, die sich lateral und oberhalb zwischen den Verarbeitungsmittel (4) und der lateralen Wand (20) und des oberen Endteils (22) der Schutzmittel (7) erstreckt.

14. Verarbeitungsbehälter (2) gemäß Anspruch 1 bis 13, **gekennzeichnet durch** Verarbeitungsmittel (4) mit einem aktiven Teil (5) im Innern des Beutels (6), der im flach gefalteten Zustand des Beutels (6) im Wesentlichen den internen Raum (23) der Schutzmittel (7) einnimmt.

15. Verarbeitungsbehälter (2) gemäß Anspruch 1 bis 14, **gekennzeichnet durch** Verarbeitungsmittel (4) mit einem aktiven Teil (5) im Innern des Beutels (6), der sich im ausgestreckten Zustand des Beutels (6) im Wesentlichen komplett im internen Raum (14) des Beutels (6) befindet, wobei die Mindesthöhe der Schutzkammer (24) gleich Null ist.

16. Verarbeitungsbehälter (2) gemäß Anspruch 1 bis 15, **gekennzeichnet durch** Verarbeitungsmittel (4) mit einem aktiven Teil (5) im Innern des Beutels (6), ggf. mit der Möglichkeit der relativen Verschiebung, **durch** die innere Seite des unteren Endteils (12) des Beutels (6) oder **durch** eine steife Platte (17), die den unteren Endteil (12) des Beutels (6) im Innern bestückt.

17. Verarbeitungsbehälter (2) gemäß Anspruch 1 bis 16, **gekennzeichnet durch** die Tatsache, dass er Mittel (31) umfasst, die geeignet sind, zu seinem Festhalten in einem Container (3) beizutragen, wobei der Beutel (6) die gewünschte Menge des wenigstens einen Produkts enthält.

18. Verarbeitungsbehälter (2) gemäß Anspruch 17, **gekennzeichnet durch** die Tatsache, dass die Mittel (31), die geeignet sind, zu seinem Festhalten in einem Container (3) beizutragen, in die Schutzmittel (7) integriert oder ihnen zugeordnet sind, wobei der Beutel (6) die gewünschte Menge des wenigstens einen Produkts enthält.

19. Mischbehälter (2), **gekennzeichnet durch** die Tatsache, dass er aus einem Behälter (2) gemäß Anspruch 1 bis 18 gebildet wird, in dem die Verarbeitungsmittel (4) Mischmittel sind, wobei der Behälter (2) wenigstens eine erste Einführöffnung (18) in den Behälter (2) eines ersten Teils des wenigstens einen Produkts, der auf dem Beutel ausgespart ist, der insbesondere an seinem unteren Endteil (12) anliegt, und wenigstens eine zweite Einführöffnung (25) eines zweiten Teils wenigstens eines Produkts, der im oberen Endteil (22) der Schutzmittel (7) ausgespart ist.

20. Verarbeitungsvorrichtung (1) wenigstens eines biopharmazeutischen Produkts, die einen Behälter (2) gemäß Anspruch 17, 18 und 19 umfasst, und dass sie von den Ansprüchen 17 und 18 abhängt, und einen steifen Container (3), der geeignet ist, den Behälter (2) aufzunehmen, dessen Beutel (6) im flach gefalteten Zustand, im ausgestreckten Zustand oder in einem Zwischenzustand ist, um es dem Beutel (6) zu ermöglichen, sich zu verformen, um von einem Zustand zum anderen überzugehen, und den Beutel (6) insbesondere seine elastische laterale Wand (11) zu führen, damit der Beutel (6) ein Volumen erhält, mit einem Volumen, das zu dem des Containers (3) komplementär ist, wenn das wenigstens eine Produkt in den Behälter (2) eingeführt wird.

21. Umsetzungsverfahren einer Verarbeitungsvorrichtung (1) gemäß Anspruch 20, in dem:
Über einen Behälter (2) und einen Container (3), die die Verarbeitungsvorrichtung bilden, verfügt wird und der Beutel (6) des Behälters im flach gefalteten Zustand in den Container (3) auf den Boden (8) platziert wird;
eine Einführöffnung (18) des Produkts des Behälters (2) offen ist, progressiv in den Behälter (2) das wenigstens eine Produkt eingeführt wird und gleichzeitig der Beutel (6) zum Ausstrecken nach oben gebracht wird und das Volumen des Containers (3) annimmt, wobei der interne Raum (14) des Beutels (6) mit steigendem Volumen das wenigstens eine Produkt aufnimmt; diese Stufe fortgeführt wird, bis in den Behälter (2) die gewünschte Menge des wenigstens einen Produkts eingeführt worden ist;
man während dieser Stufe die Schutzmittel (7) sich nach oben verschieben lässt, wobei sie durch die Verschiebung des oberen Endteils (13) des Beutels (6) angetrieben werden;
während dieser Stufe die Höhe der Schutzkammer (24) ausgehend von der maximalen extremen Höhe absinken und sich automatisch in Abhängigkeit vom Zustand des Beutels (6) anpassen gelassen wird, bis der Beutel (6) sich in Abhängigkeit von der Produktmenge, die in den Behälter (2) eingeführt wird, sich derart im entfalteten und ausgestreckten Zustand oder jedem Zwischenzustand befindet, dass die Verarbeitungsmittel (4) in allen Zuständen des Beutels (6) von der lateralen Wand (11) und der oberen Endwand (13) des Beutels (6) beabstandet sind und dabei gleichzeitig an seiner unteren Endwand (12) anliegen;
die Verarbeitungsmittel (4) des wenigstens einen, sich im Behälter (2) befindenden Produkts umgesetzt werden.

22. Verfahren gemäß Anspruch 21, **gekennzeichnet durch** die Tatsache, dass die Umsetzung der Verarbeitungsmittel (4) des wenigstens einen, sich im Behälter (2) befindenden Produkts vor, während oder nach der Einführung der gewünschten Menge des wenigstens einen Produkts in den Behälter (2) begonnen wird.

23. Mischverfahren, in dem das Verfahren gemäß Anspruch 21 und 22 umgesetzt wird, **gekennzeichnet durch** die Tatsache, dass:
über einen Mischbehälter (2) gemäß Anspruch 19 verfügt wird, dessen wenigstens eine zweite Öffnung (25) verschlossen ist;
der Beutel (6) des Behälters (2) im flach gefalteten Zustand in dem Container (3) auf dem Boden (8) platziert wird;
in den Mischbehälter (2) ein erster Teil wenigstens eines Produkts **durch** wenigstens eine zu diesem Zweck offene erste Einführöffnung (18) eingeführt wird,
in den Behälter (2) ein zweiter Teil wenigstens eines Produkts **durch** die zu diesem Zweck offene zweite Einführöffnung (25) eingeführt wird.

24. Mischverfahren gemäß Anspruch 23, **gekennzeichnet durch** die Tatsache, dass die Mischmittel (4) umgesetzt werden, während die wenigstens zweite Einführöffnung (25) geöffnet oder geschlossen ist.

25. Mischverfahren gemäß Anspruch 23 und 24, **gekennzeichnet durch** die Tatsache, dass die Mischmittel (4) bis zum kompletten oder im Wesentlichen kompletten Entleeren des Beutels (6) umgesetzt werden.
